# EUROPÄISCHE PATENTSCHRIFT

(11) **EP 3 501 400 B1**
(45) Veröffentlichungstag und Bekanntmachung des Hinweises auf die Patenterteilung: **08.06.2022**
(21) Anmeldenummer: 17209022.7
(22) Anmeldetag: 20.12.2017
(51) Int. Cl.: A61B 6/04, A61B 6/08, A61B 6/00, A61B 5/00

(54) **VERFAHREN UND VORRICHTUNG ZUR SICHERSTELLUNG EINER KORREKTEN POSITIONIERUNG FÜR EINE RADIOGRAPHIEAUFNAHME**
METHOD AND DEVICE FOR ENSURING A CORRECT POSITIONING FOR A RADIOGRAPHY RECEIVER
PROCÉDÉ ET DISPOSITIF PERMETTANT D'ASSURER UN POSITIONNEMENT CORRECT POUR UNE IMAGE RADIOGRAPHIQUE

(43) Veröffentlichungstag der Anmeldung: 26.06.2019
(73) Patentinhaber: Siemens Healthcare GmbH, 91052 Erlangen (DE)
(72) Erfinder: Jörger, Clemens, 91301 Forchheim (DE); Nanke, Ralf, 91077 Neunkirchen am Brand (DE); Oepping, Susanne, 91052 Erlangen (DE)

(56) Entgegenhaltungen:
- DE-A1-102012 201 798
- DE-A1-102015 201 070
- US-A1- 2016 089 094
- US-A1- 2017 119 338
- US-A1- 2017 322 484

## Beschreibung

Die Erfindung betrifft ein Verfahren und eine Vorrichtung zur Positionierung eines Körperbereichs für eine Aufnahme durch ein Radiographiesystem, insbesondere zum Echtzeitabgleich von Kamerabild mit simuliertem Röntgen. Die Erfindung betrifft darüber hinaus eine Steuervorrichtung für ein Radiographiesystem sowie ein entsprechendes Radiographiesystem.

Bei Radiographieaufnahmen wird das aufzunehmende Objekt, z.B. ein Knie, ein Handgelenk oder ein Fußgelenk, von einer Fachkraft so positioniert, dass für die anstehende Untersuchung eine brauchbare Aufnahme erstellt werden kann. Es kommt jedoch häufig vor, dass die Ausrichtung bzw. Positionierung des Objektes oder der aufnehmenden Elemente nicht optimal war, so dass das Objekt in der Aufnahme z.B. verschoben ist, ein Teil des Objektes abgeschnitten ist, oder das Objekt aus einem falschen Aufnahmewinkel heraus abgebildet worden ist.

Die Qualität der Positionierung wird bisher anhand des aufgenommenen Röntgenbildes beurteilt, zumeist durch die Erfahrung der Fachkraft bzw. basierend auf subjektiver Wahrnehmung. Wenn die Positionierung nicht korrekt war, muss eine weitere Aufnahme erstellt werden, was eine zusätzliche Dosis für den Patienten und mehr Zeitaufwand für die Fachkraft bedeutet. Dies stellt einen Nachteil für den Patienten und die Fachkraft dar. Zudem mindert jede zusätzliche Aufnahme die Lebensdauer des Radiographiesystems.

Bisher gibt es keine technische Lösung. Bei nicht diagnostizierbaren Bildern muss eine neue Aufnahme durchgeführt werden.

In der Druckschrift US 2017/322484 A1 wird vorgeschlagen, eine dreidimensionale Positionierungs-Aufnahme des Körperbereichs mit einem 3D-Kamerasystem zu erstellen, ein Vorschau-Bild zu erstellen, und dieses automatisch dahingehend zu untersuchen, ob es den Körperbereich im Rahmen der Untersuchungsanforderung für die Radiographieaufnahme korrekt darstellt.

Es ist eine Aufgabe der vorliegenden Erfindung, ein alternatives, komfortableres Verfahren und eine entsprechende Vorrichtung bzw. Steuereinrichtung zur Steuerung eines Radiographiesystems anzugeben, mit dem die oben beschriebenen Nachteile vermieden werden.

Diese Aufgabe wird durch ein Verfahren gemäß Patentanspruch 1, eine Vorrichtung gemäß Patentanspruch 8 sowie durch eine Steuereinrichtung gemäß Patentanspruch 10 und ein Radiographiesystem gemäß Patentanspruch 11 gelöst.

Vorab wird zu besseren Verständnis der folgenden Ausführungen klargestellt, dass unter einem Radiographiesystem im Sinne der Erfindung ein System zur Projektionsradiografie und nicht für Schnittbildverfahren (Tomographie) verstanden wird. Es werden also Bereiche des Körpers des Patienten aus einer Richtung mit Röntgenstrahlung durchstrahlt. Auf der Gegenseite wird die Strahlung mit geeigneten Materialien, z.B. Film oder einem ortsauflösenden Detektor) registriert und dadurch ein, in der Regel zweidimensionales, Projektionsbild erstellt. Systeme zur Computertomographie oder entsprechende Systeme stellen keine Radiographiesysteme im Sinne der Erfindung dar. Die Erfindung entfaltet ihre Vorteile besonders eindrucksvoll im Bereich der zweidimensionalen Radiographieaufnahmen.

Das Prinzip der Erfindung beschränkt sich dabei auf digitale Radiographie-Systeme. Bevorzugte Systeme sind diesbezüglich bodenmontierte Systeme, Systeme mit deckenmontiertem Strahler, ggf. mit Tisch und Rasterwandgerät, Systeme mit Strahler und Detektor an robotischen Tragarmen, aber auch mobile Röntgensysteme.

Das erfindungsgemäße Verfahren zur Positionierung eines Körperbereichs eines Patienten, der ein Mensch aber auch ein Tier sein kann, für eine, insbesondere zweidimensionale, Radiographieaufnahme durch ein Radiographiesystem, umfasst die folgenden Schritte:
a) Bereitstellung einer Untersuchungsanforderung.
   In diesem Schritt wird dem Verfahren mitgeteilt, um welchen Körperbereichs, z.B. welchen Knochen oder welches Organ es sich handelt und welche Aufnahme durchgeführt werden soll. Die Untersuchungsanforderung kann einfach einen Hinweis auf den Körperbereich umfassen, z.B. "Knie AP", "Knie Lateral". Zusätzlich zur Untersuchungsanforderung kann auch ein Organprogramm bereitgestellt werden, welches eine Untersuchungsanforderung umfasst. Das Organprogramm heißt z.B. Knie AP, beinhaltet aber alle notwendigen Parameter für eine spezielle Röntgenaufnahme, z.B. Informationen über den Generator, die Bildverarbeitung, die Bilddarstellung und/oder die Geräte-Position.
b) Vorpositionierung des Körperbereichs.
   In dem Radiographiesystem wird der Körperbereich für die Radiographieaufnahme vorpositioniert. Dazu wird beispielsweise der Patient so positioniert, dass der betreffende Körperbereich über einem Bilddetektor ausgerichtet wird.
c) Vorpositionierung einer Aufnahmeeinheit.
   Dazu wird die Aufnahmeeinheit und ggf. auch der Bilddetektor des Radiographiesystems für die Radiographieaufnahme passend positioniert. Diese Positionierung sollte entsprechend der Untersuchungsanforderung erfolgen. Wenn die Untersuchungsanforderung entsprechende Steuerbefehle umfasst oder das Verfahren Zugriff auf eine Datenbank mit Steuerbefehlen hat, die mit Informationen einer Untersuchungsanforderung datentechnisch verknüpft sind, kann diese Vorpositionierung durchaus automatisch erfolgen.
d) Erstellung einer Positionierungs-Aufnahme.
   Es wird eine dreidimensionale Positionierungs-Aufnahme des Körperbereichs mit einem 3D-Kamerasystem erstellt. Dieses 3D-Kamerasystem kann eine 3D-Kamera umfassen oder aber 2 Kameras, deren Bildinformationen miteinander kombiniert werden. Die Positionierungs-Aufnahme wird erstellt, während sich der Körperbereich in seiner in Schritt b) eingenommenen Position befindet. Sollte der Patient sich bewegt haben, kann auch dies als Vorpositionierung im Sinne der Erfindung verstanden werden, da Schritt b) durchaus auch vor Schritt a) oder nach Schritt c) erfolgen kann.
e) Erstellung eines Vorschau-Bildes
   Zur Erstellung des Vorschau-Bildes wird zunächst aus der Positionierungs-Aufnahme ein Patientenmodell generiert, z.B. ein dreidimensionales Körper- bzw. Knochenmodell. Aus diesem Patientenmodell wird dann das Vorschau-Bild erstellt, z.B. als Projektion eines dreidimensionalen Patentenmodells auf eine zweidimensionale Ebene bzw. als Ausschnitt aus diesem Patientenmodell. Das Vorschau-Bild gibt dabei eine Darstellung wieder, wie sie bestimmungsgemäß mit der Aufnahmeeinheit des Radiographiesystems in der in Schritt c) eingenommenen Vorpositionierung erfolgt wäre.

Der letzte Punkt, der im Grunde eine Simulation der späteren Radiographieaufnahme darstellt, ist dadurch realisierbar, dass die relative Position des 3D-Kamerasystems relativ zu der Aufnahmeeinheit bekannt ist oder zumindest sehr leicht ermittelt bzw. kalibriert werden kann. Da diese Positionen bekannt sind, kann ein Zusammenhang zwischen der Positionierungs-Aufnahme und dem Blickwinkel und Aufnahmebereich der Aufnahmeeinheit hergestellt werden. Aus Vorpositionierungsdaten der Aufnahmeeinheit und der Positionierungs-Aufnahme kann diese durch Berechnungen im Raum transformiert werden, damit sie aus dem Blickwinkel der Aufnahmeeinheit dargestellt wird. Es muss dazu im Grunde nur die Transformationsmatrix der Blickrichtung des 3D-Kamerasystems zur Blickrichtung der Aufnahmeeinheit bekannt sein. Somit kann das Patientenmodell in Position und Einblendung automatisch auf den Körperbereich ausgerichtet werden.

Das Patientenmodell kann beispielsweise ein physikalisches Modell des Objektes sein, eine Art Voxel-Phantom mit definierten Eigenschaften. Entsprechend der Aufnahmegeometrie und -parameter können Röntgenquanten simuliert werden, die das Objekt durchlaufen, dort gestreut oder absorbiert werden und am Ende ein Projektionsbild auf dem Detektor erzeugen, z.B. mittels Ray-Tracing oder Monte-Carlo-Simulation. Während dieser Simulation sollten die Parameter der Röntgenquanten den realen Parametern (denen zur Aufnahme der geplanten Radiographieaufnahme) entsprechen, insbesondere bezüglich der Strahlenergie, des Strahlstroms und/oder der Größe kV/mAs. Damit kann insbesondere eine Über- oder Unterbelichtung im Voraus erkannt werden.

### f) Ausgabe

In diesem Schritt wird das Vorschau-Bild und eine auf dem Vorschau-Bild basierende Positionierungs-Information ausgegeben. Ausserdem erfolgt eine automatische Untersuchung des Vorschau-Bildes dahingehend , ob es den Körperbereich im Rahmen der Untersuchungsanforderung für die Radiographieaufnahme korrekt darstellt.

Ein Bediener kann mit Hilfe des Vorschaubildes entscheiden, ob die Einstellungen am System bzw. die Vorpositionierungen passen oder korrigiert werden müssen. Eine Radiographieaufnahme muss dazu nicht erfolgen und der Patient bis zur Entscheidung keiner Dosis ausgesetzt werden.

Ergänzend kann das Verfahren dem Bediener mittels der Positionierungs-Information auch automatisch mitteilen, ob das Vorschaubild akzeptabel ist oder nicht. Dabei dient eine automatische Untersuchung des Vorschau-Bildes zur Generierung der Positionierungs-Information.

Werden zur Erstellung des Patientenmodells leistungsfähige Rechner verwendet, wie sie heutzutage Standard sind, so ist ein Echtzeitabgleich von Live-Kamerabildern, die in Echtzeit zu simulierten Röntgenbildern konvertiert werden, als Positionierhilfe möglich.

Zu den Schritten e) und f) würde ich eine Alternative sehen: Es wird eine Datenbank im System aufgebaut mit RöntgenBildern bei verschiedenen System-Einstellungen (Projektionen)korreliert zu der Kamera-Sicht auf das Körperteil des Patienten. Dabei ist die Kamera-Sicht die Sicht auf ein 3D Avatar des Patienten; d.h. Spezifika des aktuellen Patientenbildes wie Kleidung, Behaarung, Belichtung,... im Raum sind rausgerechnet. Diese Datenbank besteht also aus den drei Parametern Röntgenbild, Geräte-Position und Kamera-Sicht auf Avatar. Die Datenbank wächst mit jeder Röntgenaufnahme (könnte auch von Röntgenbildern von anderen Systemen ergänzt werden). Wird nun das Gerät positioniert und das Avatar-Bild gemacht, kann man in der Datenbank das korrelierende Röntgenbild suchen und anzeigen als Vorschaubild. Verbessern kann man die Methode, indem man weitere Parameter berücksichtigt: geplante Aufnahme-Parameter (KV, mAs, Dosis,...), Bild-Prozessierung, Volumen des Avatars,...

Die erfindungsgemäße Vorrichtung zur Positionierung eines Körperbereichs eines Patienten für eine Radiographieaufnahme durch ein Radiographiesystem umfasst die folgenden Komponenten:
- Eine Schnittstelle zur Bereitstellung einer Untersuchungsanforderung des Körperbereichs. Diese Schnittstelle kann z.B. eine Datenschnittstelle zu einer Eingabeeinheit darstellen, über die ein Benutzer die Untersuchungsanforderung manuell eingeben kann, z.B. einen Computer oder einfach eine Tastatur.
- Eine Schnittstelle zur Vorpositionierung einer Aufnahmeeinheit des Radiographiesystems. Diese Schnittstelle kann beispielsweise eine Bewegungseinheit steuern (oder einer Steuereinheit diesbezügliche Steuerdaten senden), welche die Aufnahmeeinheit bewegen kann.

Besonders bevorzugt umfasst die Vorrichtung auch eine Vorpositionierungs-Anzeigeeinheit, mittels der die gewünschte Positionierung des Körperbereichs angezeigt werden kann.

Des Weiteren ist bevorzugt, dass die Vorrichtung Sensoren aufweist, welche eine Positionierung eines Körperbereichs in einem Radiographiesystem messen können. Damit ließe sich überprüfen, ob ein Körperbereich entsprechend vorpositioniert worden ist.
- Ein 3D-Kamerasystem zur Erstellung einer dreidimensionalen Positionierungs-Aufnahme des Körperbereichs oder eine Datenschnittstelle zum Empfang einer solchen Positionierungs-Aufnahme. Da ein 3D-Kamerasystem nicht zwingend Teil der Vorrichtung sein muss, sondern lediglich dessen Bildaufnahme als Positionierungs-Aufnahme benötigt wird, kann theoretisch auf bereits am Radiographiesystem oder im Raum befindliche Kamerasysteme zurückgegriffen werden.
- Eine Erstellungseinheit zur Erstellung eines Vorschau-Bildes aus der Positionierungs-Aufnahme, wobei die Erstellungseinheit dazu ausgelegt ist, das Vorschau-Bild aus einem aus der Positionierungs-Aufnahme generierten Patientenmodell zu erstellen, wobei das Vorschau-Bild eine Darstellung wiedergibt, wie sie bestimmungsgemäß mit der Aufnahmeeinheit des Radiographiesystems in der in Schritt c) eingenommenen Vorpositionierung erfolgt wäre.

Bevorzugt umfasst die Vorrichtung eine separate Modellerstellungseinheit, in der das Patientenmodell erstellt wird. Im einfachsten Fall kann die Generierung des Patientenmodells auch dadurch erreicht werden, dass aus einer Datenbank entsprechend der Untersuchungsanforderung und der relativen Orientierung von 3D-Kamerasystem und Aufnahmeeinheit ein passendes Patientenmodell ausgewählt wird.
- Eine Ausgabeeinheit zur Ausgabe des Vorschau-Bildes bzw. einer auf dem Vorschau-Bild basierenden Positionierungs-Information. Zur Erstellung der Positionierungs-Information, die anzeigbare Informationen umfasst bzw. Daten für eine weitere automatische Bearbeitung durch einen Computer, findet eine automatische Untersuchung des Vorschau-Bildes dahingehend statt, ob es den Körperbereich für die Radiographieaufnahme korrekt darstellt.

Eine erfindungsgemäße Steuereinrichtung zur Steuerung eines Radiographiesystems ist für die Durchführung eines erfindungsgemäßen Verfahrens ausgelegt und/oder umfasst eine erfindungsgemäße Vorrichtung.

Ein erfindungsgemäßes Radiographiesystem umfasst eine erfindungsgemäße Steuereinrichtung.

Ein Großteil der zuvor genannten Komponenten der Vorrichtung bzw. der Steuereinrichtung können ganz oder teilweise in Form von Softwaremodulen in einem Prozessor einer entsprechenden Vorrichtung bzw. Steuereinrichtung realisiert werden. Eine weitgehend softwaremäßige Realisierung hat den Vorteil, dass auch schon bisher verwendete Rechensysteme bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Steuereinrichtung eines Radiographiesystems ladbar ist, mit Programmabschnitten, um alle Schritte des erfindungsgemäßen Verfahrens auszuführen, wenn das Programm in dem Rechensystem bzw. der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem bzw. zur Steuereinrichtung und/oder zur Speicherung an oder in dem Rechensystem bzw. der Steuereinrichtung kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Normalerweise wird als Positionierungs-Aufnahme von dem 3D-Kamerasystem ein dreidimensionales Oberflächenbild von dem Körperbereich aufgenommen oder erstellt. Auch wenn das Patientenmodell theoretisch, z.B. wie oben beschrieben, aus einer Menge vieler potentieller Modelle aus einer Datenbank ausgewählt werden kann, ist bevorzugt, dass ein dreidimensionales Patientenmodell aus dem Oberflächenbild und dreidimensionalen Organ-Datenobjekten einer Organdatenbank erzeugt wird. Die Organdatenbank enthält dabei bevorzugt dreidimensionale grafische Objekte insbesondere der Gruppe Organe, Knochen, Knorpel, Muskeln, Gefäße, Nerven und sonstiges Gewebe und zusätzlich Marker, mit denen ihre Positionierung in dem Oberflächenbild bzw. eine Registrierung mit dem Oberflächenbild möglich ist.

Aus der dreidimensionalen Oberflächeninformation kann daraus z.B. mittels des Prinzips des Machine-Learning ein Avatar berechnet werden.

Bevorzugt wird danach aus einer Kombination der Position der vorpositionierten Aufnahmeeinheit und des dreidimensionalen Patientenmodells das Vorschau-Bild erstellt. Das kann beispielsweise dadurch geschehen, dass das Patientenmodell dermaßen im Raum verschoben bzw. gedreht wird, dass es eine Ansicht wiedergibt, als wäre das 3D-Kamerasystem identisch zur Aufnahmeposition ausgerichtet. Dann wird bevorzugt eine Projektion des Patientenmodells in Blickrichtung der Aufnahmeeinheit angefertigt.

Bevorzugt umfasst eine diesbezügliche Vorrichtung eine Organdatenbank oder eine Datenschnittstelle zu einer solchen Organdatenbank, wobei die Erstellungseinheit zur Erstellung eines dreidimensionales Patientenmodells aus der Positionierungs-Aufnahme und dreidimensionalen Organ-Datenobjekten der Organdatenbank ausgelegt ist.

Bevorzugt wird das Patientenmodell basierend auf dem Prinzip des maschinellen Lernens ("Machine Learning") und/oder aus statistischen Körpermodellen erstellt. Es hat bevorzugt die Form eines (personalisierten) Avatars. Für eine optimale Echtzeitanzeige eines simulierten Röntgenbildes ist die Ausnutzung des Prinzip des maschinellen Lernens von Vorteil, da die Rechenzeit für die Erstellung des Vorschau-Bildes reduziert wird. Dafür muss im Vorfeld ein neuronales Netzwerk mit einer großen Menge von Daten, z.B. Kamera und Röntgenbildern trainiert werden.

Bevorzugt werden durch die Untersuchungsanforderung neben der Positionierung zusätzliche Aufnahmeparameter für die Aufnahmeeinheit festgelegt, z.B. Energie, Belichtungszeit, Kollimatoreinstellung. Das Vorschaubild simuliert dabei eine Radiographieaufnahme, die mit diesen Aufnahmeparametern aufgenommen worden wäre.

Bevorzugt wird das Vorschaubild an einem Bildschirm, z.B. im Raum oder auch remote, oder als Projektion, z.B. auf einer Tischoberfläche, angezeigt . Mit Bildern, welche auf das
Objekt/in die Umgebung projiziert werden, kann dem Bediener mitgeteilt werden, wie die Sollposition aussieht bzw. was zu tun ist (z.B. könnte ein Pfeil nach rechts bedeuten dass der Körperbereich nach rechts verschoben werden müsste, bis eine Anzeige z.B. grün wird). Die Sollposition ist dabei eine Position, in der das Vorschau-Bild akzeptabel ist und somit auch später die finale Radiologieaufnahme mit hoher Wahrscheinlichkeit akzeptabel wird. Akzeptabel bedeutet, dass ein Mediziner diese Aufnahme als akzeptabel im Hinblick auf eine Nutzbarkeit für die Diagnostik ansehen würde. Rein technisch kann eine Aufnahme als akzeptabel angesehen werden, wenn z.B. der untersuchte Körperbereich vollständig abgebildet und richtig positioniert ist (z.B. der Gelenkspalt bei einer Knieaufnahme sichtbar ist), ausreichend Kontrast im Bild vorliegt, sich keine irrelevante Bereiche im Strahlenfeld befinden oder ganz grundlegend der richtige Körperbereich abgebildet wird (z.B. keine links/rechts-Verwechslung vorliegt). Theoretisch kann der Patient auch selbst der Bediener sein und die Positionierung ohne Hintergrundwissen anhand der Positionierungsinformationen durchführen.

Bevorzugt enthält die Positionierungs-Information Angaben darüber, ob das Vorschau-Bild gemäß der Untersuchungsanforderung akzeptabel ist oder nicht und bevorzugt auch, welche Einstellungen angepasst werden müssten, z.B. Kollimatoreinstellungen oder Position/Rotation der Anzeigeeinheit bzw. des Körperbereichs.

Dabei wird die Ausgabe mit einer optischen und/oder akustischen Anzeige realisiert. Sie enthält Informationen in Form bildlicher Darstellungen oder Sprachbefehle, darüber, wie die Sollposition aussieht, ob die Sollposition erreicht ist und/oder Navigationsinformationen.

Alternativ umfasst die Ausgabe die Darstellung einer Anderung eines Lichtfeldes des Radiographiesystems, wobei sich bevorzugt die Helligkeit und/oder die Farbe des Lichtfeldes ändert, sobald eine korrekte Positionierung erreicht ist.

Bevorzugt erfolgt eine Steuerung des Radiographiesystems, wobei zumindest die Schritte des erfindungsgemäßen Verfahrens mit unterschiedlichen Vorpositionierungen der Aufnahmeeinheit und/oder des Körperbereichs zwei oder mehrfach durchgeführt werden. Dabei kann diese mehrfache Durchführung in einer bevorzugten Ausführung rein automatisch ablaufen, indem der Körperbereich nicht bewegt wird und lediglich die Vorpositionierung der Aufnahmeeinheit automatisch verändert wird. Selbstverständlich kann auch eine Änderung der Vorpositionierung des Detektors erfolgen. Dieser sollte stets so positioniert sein, dass der Strahl der Aufnahmeeinheit auf den Detektor fällt. Theoretisch kann auch die Vorpositionierung des Körperbereichs alleine wechseln. Diesbezüglich könnten dem Patienten beispielsweise automatisierte Navigationshilfen angegeben werden, z.B. durch eine Stimme, welche die Positionierung ansagt (z.B. "weiter nach links") oder entsprechende Zeichen, die angezeigt werden.

Die vorgenannten Verfahrensschritte werden so lange durchlaufen, bis die Positionierungs-Information eine korrekte Positionierung der Aufnahmeeinheit gemäß der Untersuchungsanforderung anzeigt und daraufhin bevorzugt automatisch eine Bildaufnahme mit der Aufnahmeeinheit angefertigt wird.

Bei unkorrekter Voreinstellung wird also erneut eine Voreinstellung vorgenommen, bzw. die bestehenden Voreinstellungen geändert und erneut eine Positionierungs-Aufnahme gemacht und automatisch ein Vorschau-Bild erzeugt und ausgegeben. Wenn das Vorschau-Bild korrekt ist, kann die Radiographieaufnahme durchgeführt werden.

Die Datenbasis für ein trainiertes neuronales Netzwerk kann kontinuierlich durch neu aufgenommene Bilder (Röntgen- und Positionierungs-Aufnahmen, bzw. alle relevanten Parameter) ergänzt werden. Die Bewertung der immer neu aufgenommenen Bilder (diagnostizierbar, nicht diagnostizierbar, oder auch Abstufungen davon) kann vom Fachpersonal manuell durchgeführt werden oder automatisch erfolgen.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine schematische Blockdarstellung eines bevorzugten Verfahrensablaufs,
Figur 2 eine schematische Darstellung der Bildung eines Patientenmodells,
Figur 3 eine grob schematische Darstellung eines bevorzugten Radiographiesystems mit einem Ausführungsbeispiel einer erfindungsgemäßen Vorrichtung zur Durchführung des Verfahrens.

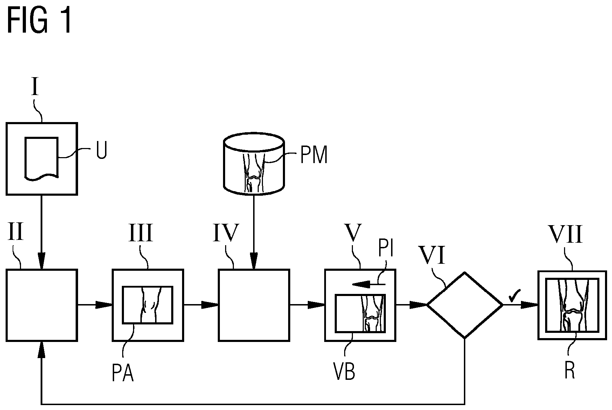

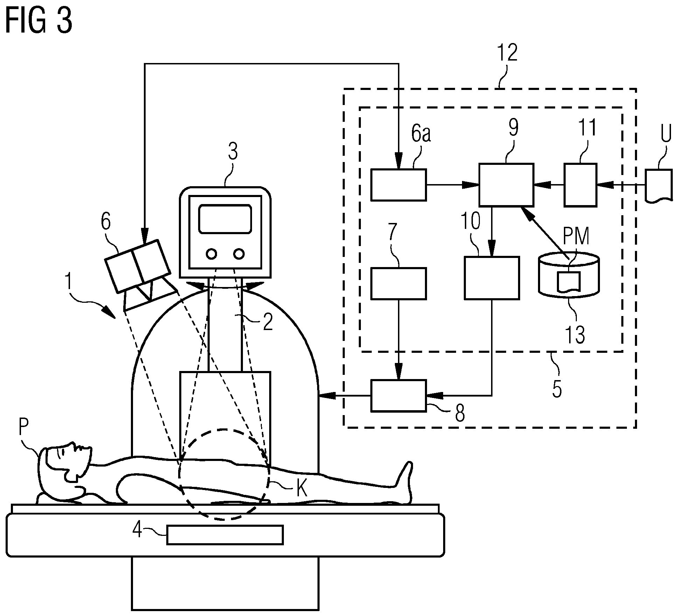

Figur 1 zeigt eine schematische Blockdarstellung eines bevorzugten Verfahrensablaufs zur Positionierung eines Körperbereichs K eines Patienten P für eine Radiographieaufnahme R durch ein Radiographiesystem 1 (s. dazu auch Fig. 3).

In Schritt I erfolgt eine Bereitstellung einer Untersuchungsanforderung U des Körperbereichs K. Hier wird dem System z.B. mitgeteilt, um welches Organ es sich handelt und welche Aufnahme durchgeführt werden soll. Im Folgenden wird der Beispielfall angenommen, dass die Untersuchungsanforderung U eine Radiographieaufnahme R des Knies zum Ziel hat.

In Schritt II erfolgt eine Vorpositionierung sowohl des Körperbereichs K in dem Radiographiesystem 1 als auch einer Aufnahmeeinheit 3 des Radiographiesystems 1 für die Radiographieaufnahme (R). Der Patient wird in dem Beispielfall gebeten, sein Knie an eine bestimmte Position zu legen, die Aufnahmeeinheit 3 wird so über dem Knie vorpositioniert, dass der Kniespalt höchstwahrscheinlich korrekt in einer Radiographieaufnahme R abgebildet werden würde. Auch wenn die Vorpositionierung von außen korrekt aussehen mag, muss dies nicht zwangsläufig zutreffen und es erfolgt eine "dosisfreie" Überprüfung gemäß der Erfindung.

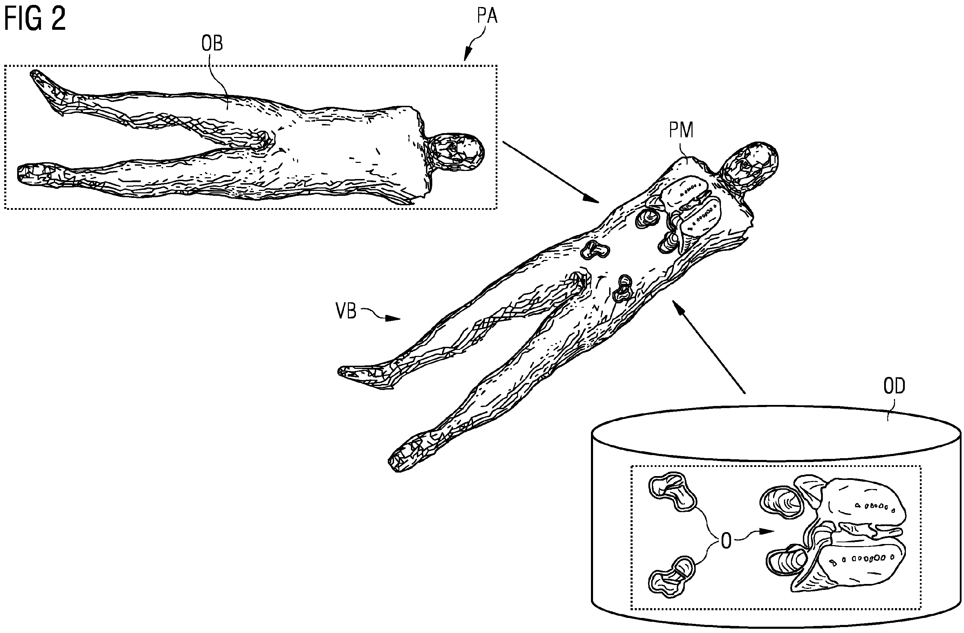

In Schritt III erfolgt die Erstellung einer dreidimensionalen Positionierungs-Aufnahme PA des Körperbereichs K mit einem 3D-Kamerasystem 6, während sich der Körperbereich K in seiner in Schritt II eingenommenen Position befindet. Man erhält also im Beispielfall ein dreidimensionales Oberflächenbild OB des Knies. Bezüglich des Oberflächenbildes OB und der Erstellung eines Patientenmodells PM wird auf die Beschreibung zu Figur 2 verwiesen.

In Schritt IV folgt die Erstellung eines Vorschau-Bildes VB aus der Positionierungs-Aufnahme PA. Zunächst wird dazu aus der Positionierungs-Aufnahme PA und ggf. auch aus der Untersuchungsanforderung U ein Patientenmodell PM generiert (s. Fig. 2). In dem Beispielfall wird ein dreidimensionales Modell des Knies mit sichtbaren Knochen und einer transparent dargestellten Haut- und Muskelschicht aus einer Datenbank entnommen und mit dem Oberflächenbild OB registriert. Das resultierende Bild stellt dann das Patientenmodell PM dar.

Danach wird das Vorschau-Bild VB, ggf. durch Anpassung der räumlichen Orientierung bzw. Positionierung des Patientenmodells PM so erstellt, dass es eine Darstellung wiedergibt, wie sie bestimmungsgemäß mit der in Schritt II vorpositionierten Aufnahmeeinheit 3 des Radiographiesystems 1 erfolgt wäre. Im Beispielfall wird eine zweidimensionale Projektion des Patientenmodells PM erstellt, die so aussieht, als wäre sie mit der Aufnahmeeinheit 3 aufgenommen worden.

In Schritt V erfolgt die Ausgabe des Vorschau-Bildes VB und einer auf dem Vorschau-Bild VB basierenden Positionierungs-Information PI. Im Beispielfall wurde bei der Verarbeitung des Vorschau-Bildes VB durch eine automatische Bilderkennung festgestellt, dass sich das Knie in einer Radiographieaufnahme zu weit rechts befinden würde. Zusätzlich zu einer grafischen Darstellung des Vorschau-Bildes VB wird ein Pfeil als Positionierungs-Information PI ausgegeben, der andeutet, dass eine Verschiebung des Knies nach links erfolgen sollte.

In der vorliegenden Ausführungsform des Verfahrens kann dies jedoch automatisch kompensiert werden.

In Schritt VI findet eine Untersuchung statt, ob eine korrekte Positionierung vorliegt. Dies ist in dem Beispielfall nicht so. Daher wird ein Teil des Verfahrens erneut von Schritt II an durchlaufen. In dem Beispielfall wird nicht das Knie nach links verschoben, sondern die Aufnahmeeinheit nach rechts und die Schritte III bis VI werden erneut durchlaufen.

Sind Aufnahmeeinheit 3 und Körperbereich K korrekt zueinander positioniert, wird in Schritt VII eine Radiographieaufnahme erstellt.

Figur 2 zeigt eine schematische Darstellung der Bildung eines Patientenmodells PM. In dem dargestellten Fall wird das Patientenmodell PM eines ganzen Korpus dargestellt. Im Grunde ist jedoch ausreichend, lediglich ein Patientenmodell PM des betreffenden Körperbereichs K anzufertigen, was z.B. der Untersuchungsanforderung U entnommen werden kann. Es ist dabei jedoch von Vorteil, zumindest einen etwas größeren Bereich im Patientenmodell PM darzustellen, als letztendlich aufgenommen werden soll, um bei Verschiebungen möglicherweise ins Bild ragende Strukturen, z.B. Organe, darstellen zu können, die eigentlich nicht durchleuchtet werden sollten.

Im oberen Kasten ist die Positionierungs-Aufnahme PA mit dem Oberflächenbild OB gezeigt, wie es mittels des 3D-Kamerasystems 6 (s. Figur 3) aufgenommen worden sein könnte. Das Oberflächenbild OB zeigt lediglich die Oberfläche des Patienten P ohne innere Strukturen. Diese Strukturen, hier als Objekte O bezeichnet, können alle möglichen inneren Strukturen im Körper umfassen, z.B. Knochen, Organe, Gefäße, Bindegewebe oder Nerven. Die Objekte liegen hier als dreidimensionale grafische Datenobjekte in einer Objektdatenbank OD vor. Bevorzugt werden Organe und Knochen aus bereits vorliegenden Modellen, z.B. aus statistischen Körpermodellen, verwendet.

Alternativ können auch dreidimensionale CT-Datensätze von vergleichbaren Patienten in das Patientenmodell PM (in den Avatar) einfügen.

Zur Erstellung des Patientenmodells PM werden die Objekte in das dreidimensionale Oberflächenbild eingepasst, z.B. mittels einer grafischen Registrierung.

Dieses Patientenmodell PM stellt die Basis zur Erstellung des Vorschau Bildes VB dar. Im einfachsten Fall kann das Vorschau-Bild VB die Abbildung des Patientenmodells PM sein, jedoch ist es vorteilhafter, wenn das Vorschau-Bild VB in seiner Art und Weise der Darstellung der geplanten Radiologieaufnahme R ähnelt oder gar entspricht. Das Vorschau-Bild VB wird bevorzugt aus dem Patientenmodell PM berechnet, indem man ein Röntgen-Projektionsbild simuliert, das sich aufgrund der Positionierung von Aufnahmeeinheit 3 und Detektor 4 zum Körperbereich K und der eingestellten Parameter wie z.B. Strahlenergie, Kollimator usw. ergeben würde. Es ist daher bevorzugt, dass das Vorschau-Bild VB eine zweidimensionale Projektion des dreidimensionalen Patientenmodells PM mit einem transparenten Oberflächenbild OB ist.

Bei den folgenden Erläuterungen wird davon ausgegangen, dass es sich bei dem Radiographiesystem 1 um ein digitales Röntgengerät handelt. Grundsätzlich ist das Verfahren aber auch an anderen Radiographiesystemen 1 einsetzbar.

Figur 3 zeigt grob schematisch ein Radiographiesystem 1 mit einer Steuereinrichtung 12 zur Durchführung des erfindungsgemäßen Verfahrens. Das Radiographiesystem 1 weist in üblicher Weise eine Aufnahmeeinheit 3 auf, welche hier eine Röntgenquelle darstellt, und während einer Radiographieaufnahme (R) einen Patienten (P) durchstrahlt, so dass die Strahlung auf einen der Aufnahmeeinheit 3 jeweils gegenüberliegenden Detektor 4 trifft. Die Aufnahmeeinheit 3 kann mittels eines Bewegungsmechanismus 2, hier einem Schwenkarm, bewegt werden, z.B. gehoben/gesenkt, verschwenkt oder verdreht werden. In der Regel erlaubt der Bewegungsmechanismus 2 mehrere unterschiedliche Bewegungen, so dass die Aufnahmeeinheit bezüglich ihrer Höhe, ihrer lateralen Position und ihres Neigungswinkels optimal bewegt werden kann.

Bei der Steuereinrichtung 12 sind nur die Komponenten dargestellt, die für die Erläuterung der Erfindung wesentlich sind. Grundsätzlich sind herkömmliche Radiographiesysteme und zugehörige Steuereinrichtungen dem Fachmann bekannt und brauchen daher nicht im Detail erläutert zu werden.

Über eine Steuerschnittstelle 8 kann das Radiographiesystem 1 von der Steuereinrichtung 12 gesteuert werden, d.h. es wird z.B. der Bewegungsmechanismus 2 zur Bewegung der Aufnahmeeinheit 3 gesteuert oder eine Radiographieaufnahme gestartet. Die Aufnahmeeinheit 3 kann auch eine Einstellung über die Steuerschnittstelle 8 erfahren z.B. hinsichtlich der Belichtungszeit oder der Strahlenergie.

Eine Benutzerschnittstelle, eine Akquisitionsschnittstelle für die aufgenommenen Bilddaten oder eine Bilddatenrekonstruktionseinheit sind nicht eingezeichnet, jedoch kann die Steuereinheit 12 selbstverständlich auch solche Elemente umfassen.

Die Steuereinrichtung 12 umfasst eine Vorrichtung 5 zur Positionierung eines Körperbereichs K eines Patienten P für eine Radiographieaufnahme R. Diese Vorrichtung 5 wirkt in dem dargestellten Fall nicht direkt auf das Radiographiesystem 1, sondern über die Steuerschnittstelle 8 der Steuereinrichtung 12.

Die Vorrichtung 5 umfasst eine Schnittstelle 11 zur Bereitstellung einer Untersuchungsanforderung U des Körperbereichs K. In dem dargestellten Fall erhält sie eine Untersuchungsanforderung U von außen, die z.B. über eine Benutzerschnittstelle eingegeben werden kann. Hierzu kann durchaus auf eine Benutzerschnittstelle der Steuereinrichtung 12 zurückgegriffen werden.

Über eine weitere Schnittstelle 7 kann eine Vorpositionierung einer Aufnahmeeinheit 3 des Radiographiesystems 1 erreicht werden. Hier sendet die Schnittstelle 7 die Daten zur Positionierung an die Steuerschnittstelle 8 der Steuereinrichtung 12 und diese steuert den Bewegungsmechanismus 2 und positioniert damit die Aufnahmeeinheit 3 gemäß den Vorgaben der Schnittstelle 7.

In dem dargestellten Fall ist ein 3D-Kamerasystem 6 nicht Teil der Vorrichtung 5, was jedoch in einer alternativen Ausführungsform durchaus der Fall sein kann. Hier jedoch greift die Vorrichtung 5 zur Erstellung einer Positionierungs-Aufnahme PA des Körperbereichs K über eine Kameraschnittstelle 6a auf ein 3D-Kamerasystem 6 zu, steuert dessen Funktion, startet eine Aufnahme und empfängt dessen dreidimensionale Positionierungs-Aufnahme PA.

Diese Positionierungs-Aufnahme PA wird dann für die Erstellungseinheit 9 bereitgestellt, wie auch die Untersuchungsanforderung U mittels der Schnittstelle 11 der Erstellungseinheit 9 bereitgestellt wird. Gemäß der Positionierungs-Aufnahme PA und ggf. auch der Untersuchungsanforderung U wird ein Patientenmodell PM generiert, indem es aus einer Datenbank 13 die hier optional Teil der Vorrichtung 5 ist, entsprechend ausgewählt wird.

Zudem wird mittels der Erstellungseinheit 9 und dem generierten Patientenmodell PM ein Vorschau-Bild VB erstellt, welches eine Darstellung wiedergibt, wie sie bestimmungsgemäß mit der Aufnahmeeinheit 3 des Radiographiesystems 1 erfolgt wäre. Dies kann beispielsweise dadurch erreicht werden, dass zunächst ein dreidimensionales Patientenmodell PM erstellt wird, dieses gemäß der Relativposition des 3D-Kamerasystem zur Aufnahmeeinheit 3 entsprechend im Raum verschoben bzw. gedreht wird und dann eine Projektion des Patientenmodells angefertigt wird. Alternativ kann natürlich auch die Projektionsrichtung entsprechend angepasst werden. In dem dargestellten Fall, bei dem das 3D-Kamerasystem gegenüber der Aufnahmeeinheit 3 sowohl verschoben als auch verkippt ist, könnte beispielsweise das Patientenmodell PM so im Raum orientiert werden, als wäre es von dem 3D-Kamerasystem am Ort der Aufnahmeeinheit 3 aufgenommen worden. Daraus kann aus einer entsprechenden vertikalen Projektion ein geeignetes Vorschaubild erstellt werden.

Über eine Ausgabeeinheit 10 kann das Vorschau-Bild VB bzw. eine auf dem Vorschau-Bild VB basierende Positionierungs-Information PI ausgegeben werden. Die Ausgabeeinheit 10 kann also ein Display sein oder eine Schnittstelle zum Datenaustausch mit einem Computer. In dem hier dargestellten Fall ist die Ausgabeeinheit 10 (auch) dazu ausgelegt, Daten an die Steuerschnittstelle 8 zu senden und damit z.B. eine weitere Vorpositionierung der Aufnahmeeinheit 3 zu erreichen.

Es wird abschließend noch einmal darauf hingewiesen, dass es sich bei den vorhergehend detailliert beschriebenen Verfahren sowie bei den dargestellten Vorrichtungen bzw. Einrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

### Bezugszeichenliste

- 1: Radiographiesystem
- 2: Bewegungsmechanismus
- 3: Aufnahmeeinheit
- 4: Detektor
- 5: Vorrichtung
- 6: 3D-Kamerasystem
- 6a: Kameraschnittstelle
- 7: Schnittstelle
- 8: Steuerschnittstelle
- 9: Erstellungseinheit
- 10: Ausgabeeinheit
- 11: Schnittstelle
- 12: Steuereinrichtung
- 13: Datenbank
- K: Körperbereich
- OB: Oberflächenbild
- OD: Objektdatenbank
- P: Patient
- PA: Positionierungs-Aufnahme
- PI: Positionierungs-Information
- PM: Patientenmodell
- R: Radiographieaufnahme
- U: Untersuchungsanforderung
- VB: Vorschau-Bild
- I - VII: Verfahrensschritte

## Patentansprüche

1. Verfahren zur Positionierung eines Körperbereichs (K) eines Patienten (P) für eine Radiographieaufnahme (R) durch ein Radiographiesystem (1), umfassend die Schritte:
a) Bereitstellung einer Untersuchungsanforderung (U) des Körperbereichs (K),
b) Vorpositionierung des Körperbereichs (K) in dem Radiographiesystem (1) für die Radiographieaufnahme (R),
c) Vorpositionierung einer Aufnahmeeinheit (3) des Radiographiesystems (1) für die Radiographieaufnahme (R),
d) Erstellung einer dreidimensionalen Positionierungs-Aufnahme (PA) des Körperbereichs (K) mit einem 3D-Kamerasystem (6),
e) Erstellung eines Vorschau-Bildes (VB) aus der Positionierungs-Aufnahme (PA), wobei zunächst aus der Positionierungs-Aufnahme ein Patientenmodell generiert wird und aus diesem Patientenmodell das Vorschau-Bild erstellt wird, und wobei das Vorschau-Bild (VB) eine Darstellung wiedergibt, wie sie bestimmungsgemäß mit der Aufnahmeeinheit (3) des Radiographiesystems (1) in der in Schritt c) eingenommenen Vorpositionierung erfolgt wäre und eine Simulation einer späteren Radiographieaufnahme darstellt,
f) Ausgabe des Vorschau-Bildes (VB) und einer auf dem Vorschau-Bild (VB) basierenden Positionierungs-Information (PI), wobei eine automatische Untersuchung des Vorschau-Bildes dahingehend durchgeführt wird, ob es den Körperbereich im Rahmen der Untersuchungsanforderung für die Radiographieaufnahme korrekt darstellt, **dadurch gekennzeichnet, dass** die Ausgabe der Positionierungs-Information (PI) mit einer optischen und/oder akustischen Anzeige realisiert wird und Informationen in Form von bildlichen Darstellungen oder Sprachbefehlen darüber enthält, wie die Sollposition aussieht und/oder Navigationsinformationen enthält, und/oder dass die Ausgabe der Positionierungs-Information (PI) eine Darstellung einer Änderung eines Lichtfeldes des Radiographiesystems (1) umfasst, wobei sich die Helligkeit und/oder die Farbe des Lichtfeldes ändert, sobald eine korrekte Positionierung erreicht ist.

2. Verfahren nach Anspruch 1, wobei als Positionierungs-Aufnahme (PA) von dem 3D-Kamerasystem ein dreidimensionales Oberflächenbild (OB) von dem Körperbereich aufgenommen oder erstellt wird, und ein dreidimensionales Patientenmodell (PM) aus dem Oberflächenbild (OB) und dreidimensionalen Organ-Datenobjekten (O) einer Organdatenbank (OD) erzeugt wird, und bevorzugt
aus einer Kombination der Position der vorpositionierten Aufnahmeeinheit (3) und des dreidimensionalen Patientenmodells (PM) das Vorschau-Bild (VB) erstellt wird.

3. Verfahren nach Anspruch 2, wobei das Patientenmodell (PM) basierend auf dem Prinzip des maschinellen Lernens und/oder auf statistischen Körpermodellen erstellt wird und bevorzugt die Form eines Avatars hat.

4. Verfahren nach einem der vorangehenden Ansprüche, wobei durch die Untersuchungsanforderung (U) neben der Positionierung zusätzliche Aufnahmeparameter für die Aufnahmeeinheit (3) festgelegt werden und das Vorschaubild (VB) eine Radiographieaufnahme (R) simuliert, die mit diesen Aufnahmeparametern aufgenommen worden wäre.

5. Verfahren nach einem der vorangehenden Ansprüche, wobei das Vorschaubild (VB) an einem Bildschirm oder als Projektion angezeigt wird.

6. Verfahren nach einem der vorangehenden Ansprüche, wobei die Positionierungs-Information (PI) Angaben darüber enthält, ob das Vorschau-Bild (VB) gemäß der Untersuchungsanforderung (U) akzeptabel ist oder nicht
und bevorzugt auch, welche Einstellungen angepasst werden müssten.

7. Verfahren nach einem der vorangehenden Ansprüche, wobei eine Steuerung des Radiographiesystems (1) erfolgt, wobei zumindest die Schritte d) - f) zusammen mit unterschiedlichen Vorpositionierungen der Aufnahmeeinheit (3) gemäß Schritt c) und/oder des Körperbereichs (K) gemäß Schritt b), so lange durchlaufen wird, bis die Positionierungs-Information (PI) eine korrekte Positionierung der Aufnahmeeinheit (3) gemäß der Untersuchungsanforderung (U) anzeigt und daraufhin bevorzugt automatisch eine Radiographieaufnahme (R) mit der Aufnahmeeinheit (3) angefertigt wird.

8. Vorrichtung (5) zur Positionierung eines Körperbereichs (K) eines Patienten (P) für eine Radiographieaufnahme (R) durch ein Radiographiesystem (1), umfassend
- eine Schnittstelle (11) zur Bereitstellung einer Untersuchungsanforderung (U) des Körperbereichs (K),
- eine Schnittstelle (7) zur Vorpositionierung einer Aufnahmeeinheit (3) des Radiographiesystems (1),
- ein 3D-Kamerasystem (6) zur Erstellung einer dreidimensionalen Positionierungs-Aufnahme (PA) des Körperbereichs (K) oder eine Datenschnittstelle (8) zum Empfang einer solchen Positionierungs-Aufnahme (PA),
- eine Erstellungseinheit (9) zur Erstellung eines Vorschau-Bildes (VB) aus der Positionierungs-Aufnahme (PA), wobei die Erstellungseinheit (9) dazu ausgelegt ist, das Vorschau-Bild (VB) aus einem aus der Positionierungs-Aufnahme (PA) generierten Patientenmodell (PM) zu erstellen, wobei das Vorschau-Bild (VB) eine Darstellung wiedergibt, wie sie bestimmungsgemäß mit der Aufnahmeeinheit (3) des Radiographiesystems (1) in der in Schritt c) eingenommenen Vorpositionierung erfolgt wäre und eine Simulation einer späteren Radiographieaufnahme darstellt,
- mindestens eine Ausgabeeinheit (10) zur Ausgabe des Vorschau-Bildes (VB) und einer auf dem Vorschau-Bild (VB) basierenden Positionierungs-Information (PI), Mittel zur automatischen
Untersuchung des Vorschau-Bildes (VB) dahingehend, ob es den Körperbereich (K) für die Radiographieaufnahme (R) korrekt darstellt , **dadurch gekennzeichnet, dass** die Ausgabe der Positionierungs-Information
mit einer optischen und/oder akustischen Anzeige realisiert wird und Informationen in Form von bildlichen Darstellungen oder Sprachbefehlen darüber enthält, wie die
Sollposition aussieht und/oder Navigationsinformationen enthält, und/oder
eine Darstellung einer Änderung eines Lichtfeldes des Radiographiesystems (1) umfasst, wobei sich die Helligkeit und/oder die Farbe des Lichtfeldes ändert, sobald eine korrekte Positionierung erreicht ist.

9. Vorrichtung nach Anspruch 8, welche eine Organdatenbank (OD) umfasst oder eine Datenschnittstelle zu einer solchen Organdatenbank (OD), wobei die Erstellungseinheit (9) zur Erstellung eines dreidimensionalen Patientenmodells (PM) aus der Positionierungs-Aufnahme (PA) und dreidimensionalen Organ-Datenobjekten (O) der Organdatenbank (OD) ausgelegt ist.

10. Steuereinrichtung (12), zur Steuerung eines Radiographiesystems, ausgelegt zur Durchführung eines Verfahrens nach einem der Ansprüche 1 bis 7 und/oder umfassend eine Vorrichtung (5) nach Anspruch 8 oder 9.

11. Radiographiesystem (1) umfassend eine Steuereinrichtung (12) nach Anspruch 10.

## Claims

1. Method for positioning a body region (K) of a patient (P) for a radiography acquisition (R) by a radiography system (1), comprising the steps:
a) providing an examination requirement (U) for the body region (K);
b) pre-positioning the body region (K) in the radiography system (1) for the radiography acquisition (R);
c) pre-positioning an acquisition unit (3) of the radiography system (1) for the radiography acquisition (R);
d) producing a three-dimensional positioning acquisition (PA) of the body region (K) using a 3D camera system (6);
e) producing a preview image (VB) from the positioning acquisition (PA), wherein first, a patient model is generated from the positioning acquisition, and the preview image is produced from this patient model, and wherein the preview image (VB) depicts a representation as it would be made using the acquisition unit (3) of the radiography system (1) as intended in the pre-positioning adopted in step c) and constitutes a simulation of a subsequent radiography acquisition;
f) outputting the preview image (VB) and/or positioning information (PI) based on the preview image (VB), wherein an automatic analysis of the preview image is performed to ascertain whether it represents the body region correctly in accordance with the examination requirement for the radiography acquisition, **characterised in that** the output of the positioning information (PI) is implemented using a visual and/or audible indicator and contains information in the form of pictorial representations or voice commands on how the setpoint position appears and/or contains navigation information, and/or the output of the positioning information (PI) comprises displaying a change in a light field of the radiography system (1), wherein the brightness and/or colour of the light field changes as soon as correct positioning is achieved.

2. Method according to claim 1, wherein the 3D camera system acquires or produces as the positioning acquisition (PA) a three-dimensional surface image (OB) of the body region, and a three-dimensional patient model (PM) is produced from the surface image (OB) and three-dimensional organ data objects (O) of an organ database (OD), and preferably
the preview image (VB) is produced from a combination of the position of the pre-positioned acquisition unit (3) and the three-dimensional patient model (PM).

3. Method according to claim 2, wherein the patient model (PM) is produced on the basis of the machine learning principle and/or on statistical body models, and preferably has the form of an avatar.

4. Method according to one of the preceding claims, wherein the examination requirement (U) specifies, in addition to the positioning, additional acquisition parameters for the acquisition unit (3), and the preview image (VB) simulates a radiography acquisition (R) that would have been acquired using these acquisition parameters.

5. Method according to one of the preceding claims, wherein the preview image (VB) is displayed on a screen or as a projection.

6. Method according to one of the preceding claims, wherein the positioning information (PI) contains information on whether or not the preview image (VB) is acceptable according to the examination requirement (U),
and preferably also what settings should be adjusted.

7. Method according to one of the preceding claims, wherein the radiography system (1) is controlled, wherein at least the steps d) - f) together with different pre-positionings of the acquisition unit (3) according to step c) and/or of the body region (K) according to step b) are cycled through until the positioning information (PI) indicates correct positioning of the acquisition unit (3) in accordance with the examination requirement (U), and thereupon a radiography acquisition (R) is produced by the acquisition unit (3) preferably automatically.

8. Apparatus (5) for positioning a body region (K) of a patient (P) for a radiography acquisition (R) by a radiography system (1), comprising:
- an interface (11) for providing an examination requirement (U) for the body region (K);
- an interface (7) for pre-positioning an acquisition unit (3) of the radiography system (1);
- a 3D camera system (6) for producing a three-dimensional positioning acquisition (PA) of the body region (K), or a data interface (8) for receiving said positioning acquisition (PA);
- a production unit (9) for producing a preview image (VB) from the positioning acquisition (PA), wherein the production unit (9) is designed to produce the preview image (VB) from a patient model (PM) generated from the positioning acquisition (PA), wherein the preview image (VB) depicts a representation as it would be made using the acquisition unit (3) of the radiography system (1) as intended in the pre-positioning adopted in step c) and constitutes a simulation of a subsequent radiography acquisition,
- at least one output unit (10) for outputting the preview image (VB) and positioning information (PI) based on the preview image (VB), means for automatic analysis of the preview image (VB) to ascertain whether it represents the body region (K) correctly for the radiography acquisition (R), **characterised in that** the output of the positioning information is implemented using a visual and/or audible indicator and contains information in the form of pictorial representations or voice commands on how the setpoint position appears and/or contains navigation information and/or comprises displaying a change in a light field of the radiography system (1), wherein the brightness and/or colour of the light field changes as soon as correct positioning is achieved.

9. The apparatus according to claim 8, comprising an organ database (OD) or a data interface to such an organ database (OD), wherein the production unit (9) is designed to produce a three-dimensional patient model (PM) from the positioning acquisition (PA) and three-dimensional organ data objects (O) of the organ database (OD).

10. Control device (12) for controlling a radiography system, designed to perform a method according to one of claims 1 to 7 and/or comprising an apparatus (5) according to claim 8 or 9.

11. Radiography system (1) comprising a control device (12) according to claim 10.

## Revendications

1. Procédé de positionnement d'une partie (K) du corps d'un patient (P) pour un enregistrement (R) radiographique par un système (1) de radiographie, comprenant les stades :
a) on se procure une demande (U) d'examen de la partie (K) du corps,
b) on prépositionne la partie (K) du corps dans le système (1) de radiographie pour l'enregistrement (R) radiographique,
c) un prépositionne une unité (3) d'enregistrement du système (1) de radiographie pour l'enregistrement (R) radiographique,
d) on établit un enregistrement (PA) tridimensionnel de positionnement de la partie (K) du corps par un système (6) d'appareil photographique en 3D,
e) on établit une image (VB) de vue préalable à partir de l'enregistrement (PA) de positionnement, dans lequel on crée d'abord un modèle de patient à partir de l'enregistrement de positionnement et on établit l'image de vue préalable à partir de ce modèle de patient, et dans lequel l'image (VB) de vue préalable redonne une représentation telle qu'elle serait produite, conformément aux prescriptions par l'unité (3) d'enregistrement du système (1) de radiographie, dans le prépositionnement pris au stade c) et représente une simulation d'un enregistrement radiographique ultérieur,
f) on émet l'image (VB) de vue préalable et une information (PI) de positionnement reposant sur l'image (VB) de vue préalable, dans lequel on effectue un examen automatique de l'image de vue préalable sur le point de savoir si elle représente correctement la partie du corps pour l'enregistrement radiographique, dans le cadre de la demande d'examen, **caractérisé en ce qu'**on réalise l'émission de l'information (PI) de positionnement par une indication optique et/ou acoustique et elle contient des informations sous la forme de représentations imagées ou d'instructions vocales sur la manière dont apparaît la position de consigne et/ou elle contient des informations de navigation et/ou **en ce que** l'émission de l'information (PI) de positionnement comprend une représentation d'une modification d'un champ lumineux du système (1) de radiographie, dans lequel la luminosité et/ou la couleur du champ lumineux se modifie dès qu'un positionnent correct est atteint.

2. Procédé suivant la revendication 1, dans lequel on enregistre ou on établit comme enregistrement (PA) de positionnement par le système d'appareil photographique en 3D, une image (OB) de surface en trois dimensions de la partie du corps et on produit un modèle (PM) en trois dimensions de patient à partir de l'image (OB) de surface et d'objets (O) en trois dimensions de données d'organe d'une base (OD) de données d'organe et, de préférence,
on établit l'image (VB) de vue préalable à partir d'une combinaison de la position de l'unité (3) d'enregistrement prépositionnée et du modèle (PM) en trois dimensions du patient.

3. Procédé suivant la revendication 2, dans lequel on établit le module (PM) du patient sur la base du principe de l'apprentissage automatique et/ou sur des modèles statistiques du corps et il a de préférence la forme d'un avatar.

4. Procédé suivant l'une des revendications précédentes, dans lequel, par la demande (U) d'examen, on fixe, outre le positionnement, des paramètres d'enregistrement supplémentaires pour l'unité (3) d'enregistrement et l'image (VB) de vue préalable simule un enregistrement (R) radiographique, qui aurait été pris avec ces paramètres d'enregistrement.

5. Procédé suivant l'une des revendications précédentes, dans lequel on affiche l'image (VB) de vue préalable sur un écran ou en projection.

6. Procédé suivant l'une des revendications précédentes, dans lequel l'information (PI) de positionnement contient des indications sur le point de savoir si l'image (VB) de vue préalable est acceptable suivant la demande (U) d'examen ou ne l'est pas et, de préférence également, sur les réglages, qui devraient être adaptés.

7. Procédé suivant l'une des revendications précédentes, dans lequel on effectue une commande du système (1) de radiographie, dans lequel on effectue au moins les stades d) à f) ensemble avec des prépositionnements différents de l'unité (3) d'enregistrement suivant le stade c) et/ou de la partie (K) du corps suivant le stade b), jusqu'à ce que l'information (PI) de positionnement indique un positionnement correct de l'unité (3) d'enregistrement conforme à la demande (U) d'examen et ensuite, de préférence automatiquement, on confectionne un enregistrement (R) radiographique par l'unité (3) d'enregistrement.

8. Installation (5) de positionnement d'une partie (K) du corps d'un patient (P) pour un enregistrement (R) radiographique par un système (1) de radiographie, comprenant
- une interface (11) pour disposer d'une demande (U) d'examen de la partie (K) du corps,
- une interface (7) de prépositionnement d'une unité (3) d'enregistrement du système (1) de radiographie,
- un système (6) d'appareil photographique en 3D, pour l'établissement d'un enregistrement (PA) de positionnement tridimensionnel de la partie (K) du corps ou une interface (8) de données de réception d'un enregistrement (PA) de positionnement de ce genre,
- une unité (9) d'établissement pour l'établissement d'une image (VB) de vue préalable à partir de l'enregistrement (PA) de positionnement, l'unité (9) d'établissement étant conçue pour établir l'image (VB) de vue préalable à partir d'un modèle (PM) de patient créé à partir de l'enregistrement (PA) de positionnement, dans laquelle l'image (VB) de vue préalable reproduit une représentation telle qu'elle serait produite, conformément aux prescriptions par l'unité (3) d'enregistrement du système (1) de radiographie, dans le prépositionnement pris dans le stade c) et représente une simulation d'un enregistrement radiographique ultérieur,
- au moins une unité (10) d'émission pour l'émission de l'image (VB) de vue préalable et d'une information (PI) de positionnement reposant sur l'image (VB) de vue préalable, des moyens d'examen automatique de l'image (VB) de vue préalable sur le point de savoir si elle représente correctement la partie (K) du corps pour l'enregistrement (R) radiographique, **caractérisé en ce qu'**on réalise l'émission de l'information de positionnement par une indication optique et/ou acoustique et elle contient des informations sous la forme de représentations imagées ou d'instructions vocales sur la manière dont apparaît la position de consigne et/ou elle contient des informations de navigation et/ou une représentation d'une modification d'un champ lumineux du système (1) de radiographie, dans lequel la luminosité et/ou la couleur du champ lumineux se modifie dès qu'un positionnent correct est atteint.

9. Installation suivant la revendication 8, qui comprend une base (OD) de données d'organe ou une interface de données avec une banque (OD) de données d'organe de ce genre, dans laquelle l'unité (9) d'établissement est conçue pour l'établissement d'un modèle (PM) de patient en trois dimensions, à partir de l'enregistrement (PA) de position et d'objets (O) de données d'organe en trois dimensions de la base (OD) de données d'organe.

10. Dispositif (12) de commande, pour la commande d'un système de radiographie, conçu pour effectuer un procédé suivant l'une des revendications 1 à 7 et/ou comprenant une installation (5) suivant la revendication 8 ou 9.

11. Système (1) de radiographie comprenant une unité (12) de commande suivant la revendication 10.
